# EUROPEAN PATENT APPLICATION

(11) **EP 4 374 787 A1**
(43) Date of publication of application: **29.05.2024**
(21) Application number: 21951042.7
(22) Date of filing: 17.11.2021
(51) Int. Cl.: A61B 5/145, A61B 5/1495, A61B 5/1455, A61B 5/00, A61B 5/05

(54) **DEVICE AND METHOD FOR DRIVING BIOMETRIC INFORMATION SENSOR BY USING ELECTROMAGNETIC WAVE METHOD**

(30) Priority: 19.07.2021 KR 20210094402
(71) Applicant: SB Solutions, Inc., Ulsan 44919 (KR)
(72) Inventor: SUNG, Namhwan, Ulju-gun Ulsan 44919 (KR)
(74) Representative: AWA Sweden AB
(86) International application number: PCT/KR2021/016850
(87) International publication number: WO 2023/003094

(57) **Abstract**

A device for driving an electromagnetic sensor according to an example embodiment may include a signal generator configured to generate a digital signal in which a signal level of at least one frequency band among a plurality of frequency bands is different from a signal level of another frequency band; a converter configured to convert the generated digital signal to an orthogonal frequency division modulation (OFDM) signal, to provide the same to a resonator assembly of the electromagnetic sensor, and to convert a signal returned from the resonator assembly back to the digital signal; a peak detector configured to detect a peak from the returned digital signal; and a processor configured to determine an analyte level of a target based on the peak.

## Description

### TECHNICAL FIELD

The following description provides technology for driving a biometric information sensor using an electromagnetic wave scheme.

### RELATED ART

Currently, due to westernization of modern people's eating habits, the number of people that suffer from so-called adult diseases, such as diabetes, hyperlipidemia, and blood clots, is increasing. A simple method of determining the severity of such adult diseases is to measure a biological component in the blood. Biocomponent measurement may determine the quantity of various components included in the blood, such as blood sugar, anemia, and blood coagulation, so even a general personal may easily determine whether a level of a specific component is in a normal range or abnormal range without a need to go to the hospital.

On of easy methods of measuring a biological component is to inject blood collected from a tip of a finger into a test strip and to quantitatively analyze an output signal using an electrochemical method or a photometric method. This method is suitable for the general public without expert knowledge since a corresponding component amount may be displayed on a measurement device.

However, since blood collection is painful, there is a need for technology to measure blood sugar in a way that does not require the blood collection or minimizes the blood collection.

### DETAILED DESCRIPTION

### TECHNICAL SUBJECT

Example embodiments provide a device and method for driving a biometric information sensor using an electromagnetic wave scheme.

### TECHNICAL SOLUTION

Provided is a device for driving an electromagnetic sensor, the device including a signal generator configured to generate a digital signal in which a signal level of at least one frequency band among a plurality of frequency bands is different from a signal level of another frequency band; a converter configured to convert the generated digital signal to an orthogonal frequency division modulation (OFDM) signal, to provide the same to a resonator assembly of the electromagnetic sensor, and to convert a signal returned from the resonator assembly back to the digital signal; a peak detector configured to detect a peak from the returned digital signal; and a processor configured to determine an analyte level of a target based on the peak.

According to an aspect, the signal generator may be configured to equalize a signal level of the generated digital signal based on a frequency response characteristic of the target.

According to another aspect, the signal generator may be configured to equalize a signal level of the generated digital signal based on a frequency response characteristic of the electromagnetic sensor.

According to still another aspect, the converter may be configured to convert the generated digital signal to the OFDM signal by converting the generated digital signal from a frequency domain signal to a time domain signal using inverse fast Fourier transform (IFFT).

According to still another aspect, the converter may be configured to generate an intermediate frequency (IF) signal by multiplying a real part and an imaginary part generated through the IFFT by sine waves with the same frequency and phase difference of 90 degrees, respectively, and to generate a single IF signal by adding the real part and the imaginary part of the generated IF signal.

According to still another aspect, the converter may be configured to convert the converted digital signal returned from the resonator assembly to a baseband signal by multiplying the converted digital signal by sine waves with the same frequency and phase difference of 90 degrees and by dividing the same into a real part and an imaginary part.

According to still another aspect, the converter may be configured to convert the baseband signal to a frequency domain signal using fast Fourier transform (FFT).

According to still another aspect, the device for deriving the electromagnetic sensor may further include a band pass filter configured to remove a harmonic component from the OFDM signal.

According to still another aspect, the peak determinator may be configured to find a frequency corresponding to a maximum point or a minimum point in the returned digital signal and to determine the found frequency as a resonant frequency of a sensor.

According to still another aspect, the processor may be configured to find and output a value of an analyte level that maps the determined resonant frequency from a mapping table in which resonant frequencies according to values of analyte levels are mapped.

According to still another aspect, the processor may be configured to compensate for the determined analyte level according to a surrounding environment.

Provided is a method of driving an electromagnetic sensor, the method including generating a digital signal in which a signal level of at least one frequency band among a plurality of frequency bands is different from a signal level of another frequency band; converting the generated digital signal to an OFDM signal and providing the same to a resonator assembly of an electromagnetic sensor; converting a signal returned from the resonator assembly back to the digital signal; detecting a peak from the returned digital signal; and determining an analyte level of a target based on the peak.

### EFFECT

According to some example embodiments, there may be provided a device and method for driving a biometric information sensor using an electromagnetic wave scheme.

### BRIEF DESCRIPTION OF DRAWINGS

FIG. 1 illustrates a frequency scanning scheme.
FIG. 2 illustrates a digital frequency scanning scheme according to an example embodiment.
FIG. 3 is a graph showing orthogonal frequency modulation (OFDM) according to an example embodiment.
FIG. 4 is a graph showing an example of a resonance characteristic of an electromagnetic sensor according to an example embodiment;
FIG. 5 is a graph showing an example of a frequency characteristic inside a living body according to an example embodiment.
FIG. 6 is a graph showing an example of distorted sensor output.
FIG. 7 is a graph showing an example of a frequency characteristic of a signal adjusted by an equalizer according to an example embodiment.
FIG. 8 is a graph showing an example of sensor output in which a frequency characteristic inside a living body is removed according to an example embodiment.
FIG. 9 illustrates an example of a configuration of a device for driving an electromagnetic sensor according to an example embodiment.
FIG. 10 illustrates a symbol according to an example embodiment.
FIG. 11 illustrates an example of a configuration of a device further including a band pass filter according to an example embodiment.
FIG. 12 illustrates an example of an internal configuration of an electromagnetic sensor according to an example embodiment.
FIG. 13 is a block diagram illustrating an electromagnetic sensor driving device according to an example embodiment.
FIG. 14 is a flowchart illustrating an example of an electromagnetic sensor driving method according to an example embodiment.

### BEST MODE

Specific structural or functional description related to example embodiments is provided only for examples and may be implemented through modification in various forms. Therefore, an actual implementation form is not limited to a disclosed specific example embodiment. Rather, the scope of the present specification includes modifications, equivalents, and substitutions included in technical spirit described with the example embodiments.

Although the terms "first," "second," etc., may be used herein to describe various components, the terms are only used to distinguish one component from another component. For example, a first component may be termed a second component and, likewise, a second component may also be termed a first component, without departing from the scope of this disclosure.

When a component is referred to as being "connected to" another component, it should be understood that the component may be directly connected to or accessed to the other component, but there may be still an intervening component.

The singular forms "a," "an," and "the," are intended to include the plural forms as well, unless the context clearly indicates otherwise. It will be further understood that the terms "comprises/comprising (incudes/including)," and "has/having" when used in this specification, specify the presence of stated features, integers, steps, operations, elements, and/or components, but do not preclude the presence or addition of one or more other features, integers, steps, operations, elements, components, and/or groups, thereof.

Unless otherwise defined, all terms including technical and scientific terms used herein have the same meaning as commonly understood by one of ordinary skill in the art to which example embodiments belong. Terms, such as those defined in commonly used dictionaries, should be interpreted as having a meaning that is consistent with their meaning in the context of the relevant art and/or this disclosure, and should not be interpreted in an idealized or overly formal sense unless expressly so defined herein.

Hereinafter, example embodiments will be described with reference to the accompanying drawings. In describing with reference to the accompanying drawings, like reference numerals refer to like elements throughout and repeated description related thereto is omitted.

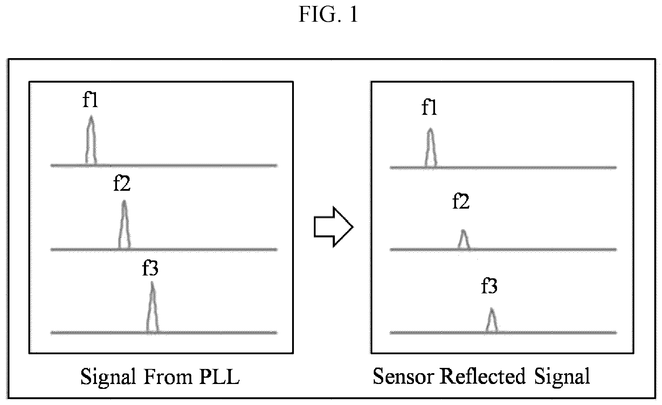

FIG. 1 illustrates a frequency scanning scheme.

Since a conventional scanning scheme using a phase lock loop (PLL) measures response to a single frequency at a time, it takes a long time to scan the entire band and an environmental change over time directly affects measurement results. Also, it is difficult to generate signals with the same magnitude at all frequencies.

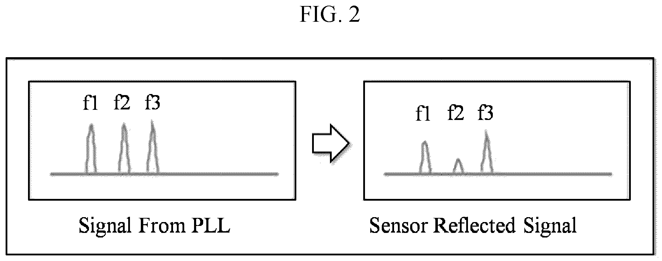

FIG. 2 illustrates a digital frequency scanning scheme according to an example embodiment.

The example embodiment may employ a digital frequency scanning scheme of measuring sensor response by simultaneously generating all frequency components in a band desired to measure. Since all frequency components are generated at a time, a measurement time may be dramatically reduced and effect of noise or environmental change over time may be minimized. Also, since a frequency is generated using the digital frequency scanning scheme, a signal with constant magnitude may be generated at all frequencies.

When simultaneously generating adjacent frequency signals, the respective components interfere with each other. To solve this, an orthogonal frequency division modulation (OFDM) technique may be used. OFDM refers to technology for modulating a transmission signal to hundreds or more orthogonal narrowband subcarriers. Using this, the respective subcarriers do not affect each other, the same result as each generating an orthogonal frequency signal may be acquired.

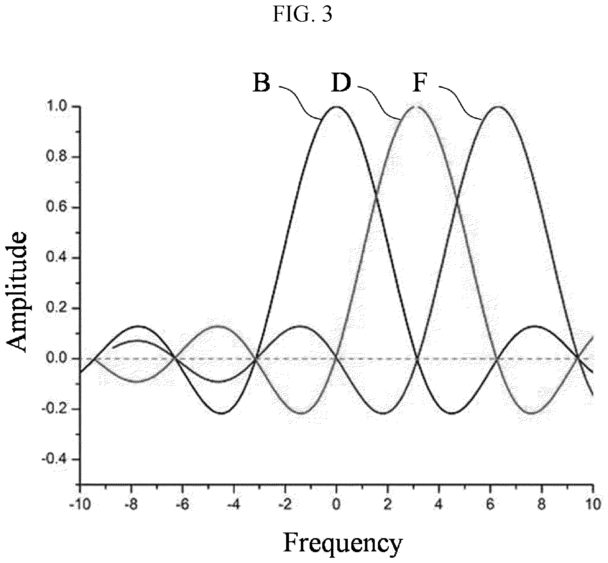

FIG. 3 is a graph showing OFDM according to an example embodiment.

Referring to FIG. 3, it can be seen that each subcarrier shows maximum amplitude at its own frequency and has amplitude of 0 at frequencies of other subcarriers, so does not affect other subcarriers.

To generate signals that do not interfere with each other, an OFDM technique may use fast Fourier transform (FFT) and inverse fast Fourier transform (IFFT). The IFFT may convert a frequency domain signal to a time domain signal and the FFT may convert the time domain signal to the frequency domain signal, and both may be used to generate OFDM signals that do not interfere with each other.

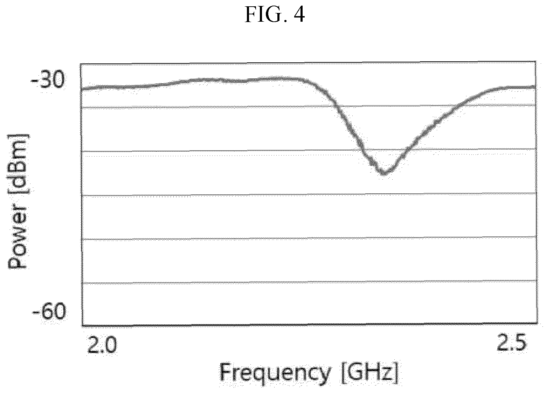

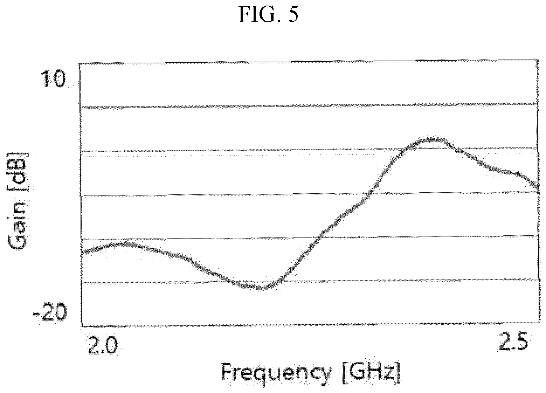

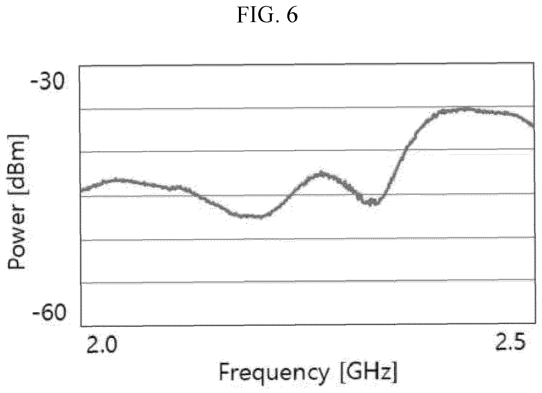

FIG. 4 is a graph showing an example of a resonance characteristic of an electromagnetic sensor according to an example embodiment, FIG. 5 is a graph showing an example of a frequency characteristic inside a living body according to an example embodiment, and FIG. 6 is a graph showing an example of distorted sensor output.

To accurately read a resonance characteristic of a sensor, an input signal to the sensor needs to have the same magnitude for all frequencies. However, referring to FIGS. 4 to 6, although a signal with the same magnitude is generated, a frequency characteristic inside a living body is added and the input signal to the sensor does not have the same magnitude for all frequencies and an output signal of the sensor is distorted accordingly. The inside of the living body has its own frequency response characteristic. Therefore, if the frequency response characteristic is not removed, it may be added with the resonance characteristic of the sensor during measurement and measurement results may be distorted. FIG. 6 shows a waveform in which sensor output is distorted due to addition of the frequency characteristic inside the living body.

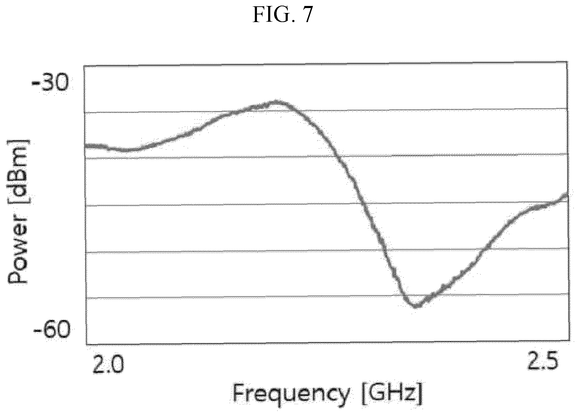

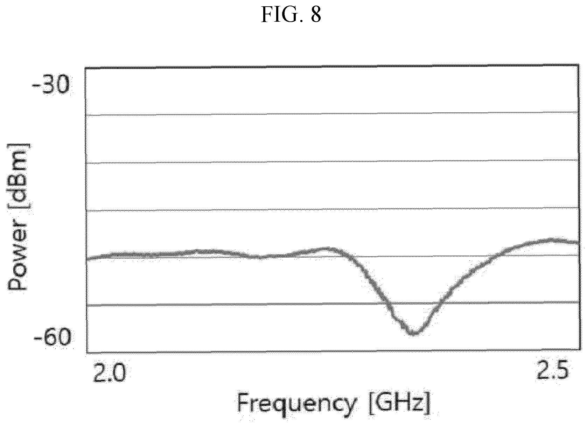

FIG. 7 is a graph showing an example of a frequency characteristic of a signal adjusted by an equalizer according to an example embodiment, and FIG. 8 is a graph showing an example of sensor output in which a frequency characteristic inside a living body is removed according to an example embodiment.

To solve an issue in which sensor output is distorted due to addition of a frequency characteristic inside a living body, a biometric information sensor driving device according to the example embodiment may include an equalizer configured to compensate for the frequency characteristic inside the living body. The equalizer verifies the frequency characteristic inside the living body in advance and differently sets magnitude of each frequency component such that the frequency characteristic may be corrected. Referring to FIGS. 7 and 8, it can be seen that the equalizer removes distortion of the sensor output by correcting the frequency characteristic inside the living body.

The equalizer according to an example embodiment may acquire a calibration curve as follows. Initially, 1) biometric information in the body (e.g., blood sugar) is measured with a biometric information measurement device through blood collection. 2) Sensor output for signal output not equalized is measured. 3) The frequency characteristic inside the living body may be acquired by finding difference between a measured sensor output waveform and a resonance characteristic waveform of a sensor corresponding to the measured biometric information. 4) The calibration curve of the equalizer may be acquired by inverting the acquired frequency characteristic inside the living body and by multiplying the same by a predefined coefficient.

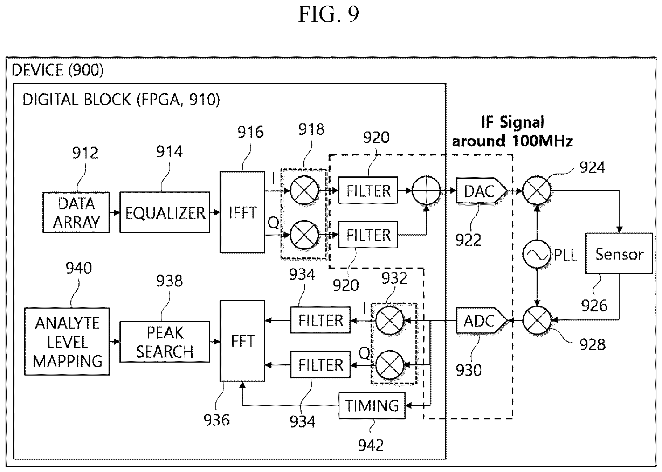

FIG. 9 illustrates an example of a configuration of a device for driving an electromagnetic sensor according to an example embodiment. A device 900 according to the example embodiment may include a digital block 910 and a sensor 926. Here, the digital block may be implemented in a form of, for example, a field programmable gate array (FPGA).

A data array block 912 may generate a data array with a regular size in a band desired to measure. For example, in the case of performing measurement in units of 10 kHz in the frequency range of 20 MHz, 20 MHz/10 kHz=2000 data arrays are required. Since FFT may be most efficiently implemented on 2^N data arrays, 2048 data arrays that is 2^N larger than 2000 may be used. Among 2048 data arrays, required 2000 data may be set to 1 and the rest may be set to 0. Accordingly, a value of a data array may be provided in a form in which a center is filled with 1 and left and right are filled with 0. A value of an actual set data array may be D(0)=0, D(1)=0, D(2)=0, ... D(21)=0, D(22)=0, D(23)=0, D(24)=1, D(25)=1, D(26)=1, ... D(2022)=1, D(2023)=1, D(2024)=0, D(2025)=0, D(2026)=0, D(2027)=0, ..., D(2045)=0, D(2046)=0, D(2047)=0. Here, D(1024) may correspond to a DC (frequency 0 Hz) component, D(0) ~ D(1023) may correspond to frequency -10.24 MHz ~ -0.01MHz, and D(1025) ~ D(2047) may correspond to frequency 0.01 MHz - 10.23 MHz.

An equalizer block 914 may generate an input signal to an IFFT block 916 by reflecting a frequency characteristic inside a living body to a uniform signal generated by the data array block 912. Here, the equalizer block 914 may equalize a signal level of a digital signal based on a frequency response characteristic of a target or a frequency response characteristic of the sensor 926.

The IFFT block 916 may convert an input frequency domain signal to a time domain signal. This signal may include each frequency component set above and may be output as a real part (I) and an imaginary part (Q).

An intermediate frequency (IF) up-converter 918 may generate an IF signal by multiplying the real part (I) and the imaginary part (Q) generated through the IFFT block 916 by sine waves with the same frequency and phase difference of 90 degrees, respectively. Here, a frequency of the multiplied sine wave may be a center frequency of the IF signal. If the frequency of the sine wave is set to 120 MHz, the generated IF signal may be a signal with 10 MHz band left and right centered on 120MHz.

The above generated IF signal may include various harmonic components in addition to the 120 MHz signal. A filter 920 is used to remove the harmonic component. A low pass filter and a band pass filter may be used as the filter 920. A cut-off frequency may be set to effectively remove the harmonic component.

A single signal may be generated by adding the real part (I) and the imaginary part (Q) of the generated IF signal and may be output through a digital-to-analog converter (DAC) 922.

A transmitting-side IF signal output through the DAC 922 may be converted to a signal of a radio frequency (RF) band in which the sensor 926 operates through an RF up-converter 924. Here, a center frequency of an RF signal may be determined with a sum of an IF frequency and a local PLL frequency of the RF up-converter 924. If a transmitting-side IF frequency is 120 MHz and a local PLL frequency is 2.4 GHz, a center frequency of an RF band becomes 0.12GHz+2.4GHz=2.52GHz.

The generated RF signal may be input to the sensor 926 and a signal with magnitude of each frequency component varying according to a resonance characteristic of the sensor 926 may be output.

The signal output from the sensor 926 may be converted to a receiving-side IF signal through an RF down-converter 928. In a configuration of the device according to the example embodiment, the RF down-converter 928 may use the same local PLL frequency as the RF up-converter 924 and thus, the receiving-side IF signal and the transmitting-side IF signal may have the same frequency band. That is, if the transmitting-side IF frequency is 120 MHz, the transmitting-side IF frequency may also be 120 MHz. However, the transmitting-side IF frequency and the receiving-side IF frequency may be differently designed depending on example embodiments. In this case, the local PLL frequency of the RF up-converter 924 and the local PLL frequency of the RF down-converter 928 may be differently set.

The receiving-side IF signal may be converted to the digital signal through an analog-to-digital converter (ADC) 930 and input back to the digital block 910.

A first input terminal of the digital block 910 is an IF down-converter 932. The IF down-converter 932 may divide the input IF signal into a real part (I) and an imaginary part (Q) and may convert the same to a baseband signal. Here, similar to the IF up-converter 918, the IF down-converter 932 may use two sine waves with the same frequency and phase difference of 90 degrees. Here, a frequency of the sine wave may use the same frequency as the receiving-side IF signal.

When passing through the IF down-converter 932, a harmonic component may be added to a signal and a filter 934 may be used to remove the harmonic component. For example, a low pass filter may be used as the filter 934.

The baseband signal generated in this manner may be converted to a frequency domain signal through an FFT block 936.

The frequency domain signal generated through the FFT block 936 may be input to a peak search block 938 configured to find a resonant frequency and to compute biometric information (e.g., blood sugar).

The peak search block 938 may determine the resonant frequency by finding a peak point of the received frequency domain signal. The received signal may be a signal with a value of each frequency component varying according to the resonance characteristic of the sensor 926 while passing through the sensor 926. The received signal may have a maximum or minimum data value at the resonant frequency of the sensor 926 depending on the configuration of the sensor 926. The peak search block 938 may find a frequency corresponding to a maximum point or a minimum point in the received signal and may determine the found frequency as the resonant frequency of the sensor 926.

When the resonant frequency of the sensor 926 is determined, an analyte level mapping block 940 may compute a value of an analyte level (e.g., blood sugar value) corresponding to the determined frequency. The analyte level mapping block 940 may have a mapping table in which resonant frequencies according to values of analyte levels are mapped and, when the resonant frequency is input, may find and output a value of an analyte level corresponding to the input resonant frequency. The analyte level mapping block 940 may additionally have a separate table or a computation formula for compensation according to a surrounding environment, such as temperature.

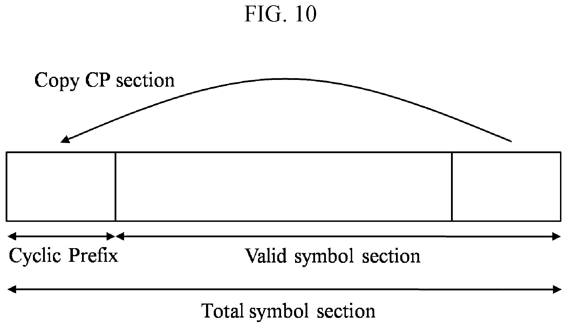

FIG. 10 illustrates a symbol according to an example embodiment.

In the digital block 910, a time difference may occur between an output signal and an input signal, which may cause a problem in signal detection. In this case, synchronization of a received signal may be found by adding a timing block 942. To this end, when outputting a transmission signal, the timing block 942 may insert a cyclic prefix (CP) that copies a last certain section of the transmission signal to the beginning and initially outputs the same. Then, when the CP is found by comparing a repetitive section of the received signal, a receiving terminal may be aware of a starting point of actual data. A CP structure is as shown in FIG. 10. When the CP is used, a data transmission time may increase by a length of a CP section, but signal detection on a receiving side may become easier.

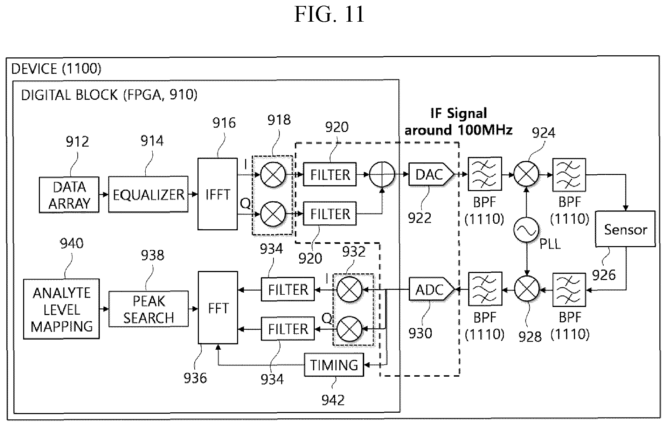

FIG. 11 illustrates an example of a configuration of a device further including a band pass filter according to an example embodiment. A device 1100 according to the example embodiment may include all the components described above with reference to FIG. 9 and, in this state, may additionally add band pass filters (BPFs) 1110 in front and behind the RF up-converter 924 and in front and behind the RF down-converter 928 to remove noise and a harmonic component, thereby improving performance.

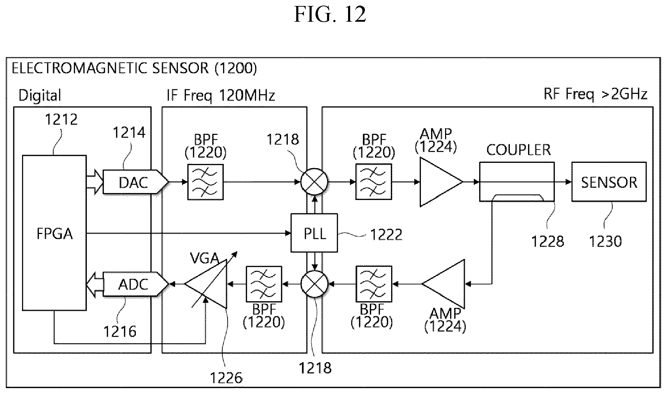

FIG. 12 illustrates an example of an internal configuration of an electromagnetic sensor according to an example embodiment. An example of a hardware configuration of an electromagnetic sensor 1200 according to the example embodiment is as shown in FIG. 12.

An FPGA 1212 may be responsible for generating and detecting an IF signal. Algorithm processing of an equalizer may also be performed in the FPGA 1212.

A DAC 1214 may convert a digital signal generated by the FPGA 1212 to an analog signal and may output the same. An ADC 1216 may convert the input analog signal to the digital signal and may deliver the same to the FPGA 1212.

A mixer 1218 may convert an IF frequency to an RF frequency or may convert the RF frequency to the IF frequency. BPFs 1220 may be located before and after the mixer 1219 and may remove a harmonic component.

A PLL 1222 may generate a reference frequency of the mixer 1218.

An amplifier (AMP) 1224 may amplify a signal in each part.

A variable gain amplifier (VGA) 1226 may prevent the ADC 1216 from being saturated by maintaining magnitude of an input signal at a certain level. Gain of the VGA 1226 may be changed under control of the FPGA 1212.

A coupler 1228 may separate an input signal and an output signal of a sensor 1230. A Tx path signal may be input to the sensor 1230 through the coupler 1228 and a signal reflected from the sensor 1230 may be delivered to an Rx path through the coupler 1228.

As time passes after inserting the sensor 1230 into the body, a resonant frequency of the sensor 1230 may gradually move and deviate from an initial frequency detection range. To correct this, the detection range may be adjusted by controlling a frequency of reference PLL of the mixer 1218.

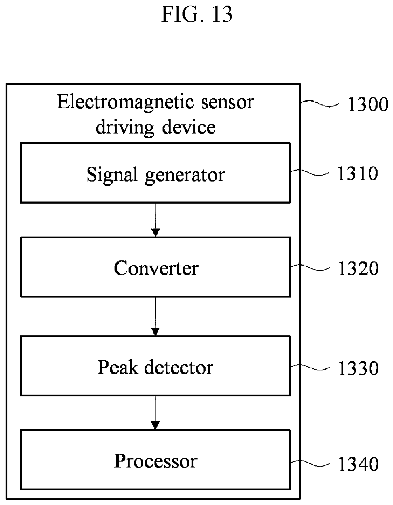

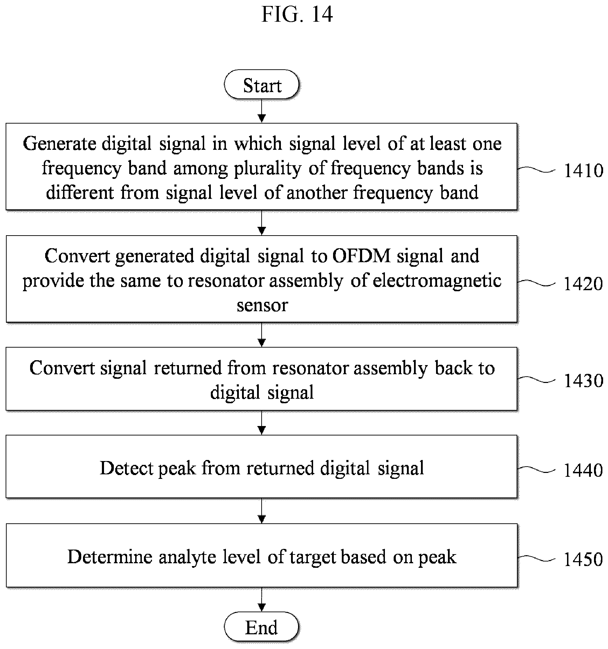

FIG. 13 is a block diagram illustrating an electromagnetic sensor driving device according to an example embodiment, and FIG. 14 is a flowchart illustrating an example of an electromagnetic sensor driving method according to an example embodiment. The electromagnetic sensor driving method according to the example embodiment may be performed by the electromagnetic sensor driving device. Referring to FIG. 13, an electromagnetic sensor driving device 1300 may include a signal generator 1310, a converter 1320, a peak detector 1330, and a processor 1340.

In operation 1410, the signal generator 1310 may generate a digital signal in which a signal level of at least one frequency band among a plurality of frequency bands is different from a signal level of another frequency band. Here, the signal generator 1310 may include the data array block 912 described above with reference to FIGS. 9 and 11. Depending on example embodiments, the signal generator 1310 may further include the equalizer block 914 described above with reference to FIGS. 9 and 11. In this case, the signal generator 1310 may equalize a signal level of the generated digital signal based on a frequency response characteristic of a target or a frequency response characteristic of an electromagnetic sensor. Equalizing may be used to compensate for a frequency characteristic inside a living body.

In operation 1420, the converter 1320 may convert the generated digital signal to an OFDM signal, and may provide the same to a resonator assembly of the electromagnetic sensor. For example, the converter 1320 may include the IFFT block 916 described above with reference to FIGS. 9 and 11. In this case, the converter 1320 may convert the generated digital signal to the OFDM signal by converting the generated digital signal from a frequency domain signal to a time domain signal using IFFT. Depending on example embodiments, the converter 1320 may further include the IF up-converter 918 described above with reference to FIGS. 9 and 11. That is, the converter 1320 may generate an IF signal by multiplying a real part and an imaginary part generated through the IFFT by sine waves with the same frequency and phase difference of 90 degrees, respectively, and may generate a single IF signal by adding the real part (I) and the imaginary part (Q) of the generated IF signal. The generated IF signal may be provided to a resonator assembly of the electromagnetic sensor. Here, the resonator assembly may correspond to the sensor 926 described above with reference to FIGS. 9 and 11. As described above, the IF signal may be delivered to the sensor 926 through the DAC 922, the RF up-converter 924, and/or the BPF 1110. The BPF 1110 may be used to remove a harmonic component from the OFDM signal.

In operation 1430, the converter 1320 may convert a signal returned from the resonator assembly back to the digital signal. For example, the converter 1320 may further include the IF down-converter 932 described above with reference to FIGS. 9 and 11. In this case, the converter 1320 may convert the converted digital signal returned from the resonator assembly to a baseband signal by multiplying the converted digital signal by sine waves with the same frequency and phase difference of 90 degrees and by dividing the same into a real part and an imaginary part. Also, the converter 1320 may further include the FFT module 936 described above with reference to FIGS. 9 and 11. In this case, the converter 1320 may convert the baseband to a frequency domain signal using FFT.

In operation 1440, the peak determinator 1330 may detect a peak from the returned digital signal. For example, the peak determinator 1330 may find a frequency corresponding to a maximum point or a minimum point in the returned digital signal and may determine the found frequency as a resonant frequency of a sensor. Here, the sensor may correspond to the sensor 926 described above with reference to FIGS. 9 and 11.

In operation 1450, the processor 1340 may determine an analyte level of the target based on the peak. For example, the processor 1340 may find and output a value of an analyte level that maps the determined resonant frequency from a mapping table in which resonant frequencies according to values of analyte levels are mapped. Depending on example embodiments, the processor 1340 may compensate for the determined analyte level according to a surrounding environment, such as temperature.

As described above, according to example embodiments of the present invention, there may be provided a device and method for driving a biometric information sensor using an electromagnetic wave scheme.

The example embodiments described herein may be implemented using hardware components, software components, and/or combination of the hardware components and the software components. For example, the apparatuses, the methods, and the components described herein may be implemented using one or more general-purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of responding to and executing instructions in a defined manner. A processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that the processing device may include multiple processing elements and/or multiple types of processing elements. For example, the processing device may include multiple processors or a processor and a controller. In addition, different processing configurations are possible, such as parallel processors.

The software may include a computer program, a piece of code, an instruction, or some combinations thereof, for independently or collectively instructing or configuring the processing device to operate as desired. Software and/or data may be permanently or temporarily embodied in any type of machine, component, physical equipment, virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more computer readable storage mediums.

The methods according to the example embodiments may be implemented in a form of program instructions that may be performed through various computer methods and recorded in computer-readable media. Also, the media may include, alone or in combination with the program instructions, data files, data structures, and the like. Program instructions stored in the media may be those specially designed and constructed for the example embodiments, or they may be well-known and available to those having skill in the computer software arts. Examples of the media include magnetic media such as hard disks, floppy disks, and magnetic tapes; optical media such as CD ROM disks and DVDs; magnetooptical media such as floptical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter.

The hardware device described above may be configured to operate as at least one software module to perform operations of the example embodiments, or vice versa.

### MODE

Although the example embodiments are described with reference to some accompanying drawings, it will be apparent to one of ordinary skill in the art that various alterations and modifications in form and details may be made in these example embodiments without departing from the spirit and scope of the claims and their equivalents. For example, suitable results may be achieved if the described techniques are performed in different order, and/or if components in a described system, architecture, device, or circuit are combined in a different manner, and/or replaced or supplemented by other components or their equivalents.

Therefore, other implementations, other example embodiments, and equivalents of the claims are to be construed as being included in the claims.

## Claims

1. A device for driving an electromagnetic sensor, the device comprising:
a signal generator configured to generate a digital signal in which a signal level of at least one frequency band among a plurality of frequency bands is different from a signal level of another frequency band;
a converter configured to convert the generated digital signal to an orthogonal frequency division modulation (OFDM) signal, to provide the same to a resonator assembly of the electromagnetic sensor, and to convert a signal returned from the resonator assembly back to the digital signal;
a peak detector configured to detect a peak from the returned digital signal; and
a processor configured to determine an analyte level of a target based on the peak.

2. The device of claim 1, wherein the signal generator is configured to equalize a signal level of the generated digital signal based on a frequency response characteristic of the target.

3. The device of claim 1, wherein the signal generator is configured to equalize a signal level of the generated digital signal based on a frequency response characteristic of the electromagnetic sensor.

4. The device of claim 1, wherein the converter is configured to convert the generated digital signal to the OFDM signal by converting the generated digital signal from a frequency domain signal to a time domain signal using inverse fast Fourier transform (IFFT).

5. The device of claim 4, wherein the converter is configured to generate an intermediate frequency (IF) signal by multiplying a real part and an imaginary part generated through the IFFT by sine waves with the same frequency and phase difference of 90 degrees, respectively, and to generate a single IF signal by adding the real part and the imaginary part of the generated IF signal.

6. The device of claim 1, wherein the converter is configured to convert the converted digital signal returned from the resonator assembly to a baseband signal by multiplying the converted digital signal by sine waves with the same frequency and phase difference of 90 degrees and by dividing the same into a real part and an imaginary part.

7. The device of claim 6, wherein the converter is configured to convert the baseband signal to a frequency domain signal using fast Fourier transform (FFT).

8. The device of claim 1, further comprising:
a band pass filter configured to remove a harmonic component from the OFDM signal.

9. The device of claim 1, wherein the peak determinator is configured to find a frequency corresponding to a maximum point or a minimum point in the returned digital signal and to determine the found frequency as a resonant frequency of a sensor.

10. The device of claim 9, wherein the processor is configured to find and output a value of an analyte level that maps the determined resonant frequency from a mapping table in which resonant frequencies according to values of analyte levels are mapped.

11. The device of claim 1, wherein the processor is configured to compensate for the determined analyte level according to a surrounding environment.

12. A method of driving an electromagnetic sensor, the method comprising:
generating a digital signal in which a signal level of at least one frequency band among a plurality of frequency bands is different from a signal level of another frequency band;
converting the generated digital signal to an orthogonal frequency division modulation (OFDM) signal and providing the same to a resonator assembly of an electromagnetic sensor;
converting a signal returned from the resonator assembly back to the digital signal;
detecting a peak from the returned digital signal; and
determining an analyte level of a target based on the peak.

13. The method of claim 12, wherein the generating comprises equalizing a signal level of the generated digital signal based on a frequency response characteristic of the target.

14. The method of claim 12, wherein the generating comprises equalizing a signal level of the generated digital signal based on a frequency response characteristic of the electromagnetic sensor.

15. The method of claim 12, wherein the providing comprises converting the generated digital signal to the OFDM signal by converting the generated digital signal from a frequency domain signal to a time domain signal using inverse fast Fourier transform (IFFT).

16. The method of device 15, wherein the providing comprises generating an intermediate frequency (IF) signal by multiplying a real part and an imaginary part generated through the IFFT by sine waves with the same frequency and phase difference of 90 degrees, respectively, and generating a single IF signal by adding the real part and the imaginary part of the generated IF signal.

17. The method of claim 12, wherein the converting comprises converting the converted digital signal returned from the resonator assembly to a baseband signal by multiplying the converted digital signal by sine waves with the same frequency and phase difference of 90 degrees and by dividing the same into a real part and an imaginary part.

18. The method of claim 17, wherein the converting comprises converting the baseband signal to a frequency domain signal using fast Fourier transform (FFT).

19. The method of claim 12, wherein the detecting comprises finding a frequency corresponding to a maximum point or a minimum point in the returned digital signal and determining the found frequency as a resonant frequency of a sensor.

20. The method of claim 19, wherein the determining comprises finding and outputting a value of an analyte level that maps the determined resonant frequency from a mapping table in which resonant frequencies according to values of analyte levels are mapped.
